(19) Europäisches Patentamt
European Patent Office
Office européen des brevets

(11) **EP 4 691 449 A1**

(12) **EUROPEAN PATENT APPLICATION**
published in accordance with Art. 153(4) EPC

(43) Date of publication:
**11.02.2026 Bulletin 2026/07**

(21) Application number: **24779083.5**

(22) Date of filing: **01.03.2024**

(51) International Patent Classification (IPC):
**A61K 6/77** *(2020.01)*      **A61K 6/836** *(2020.01)*

(52) Cooperative Patent Classification (CPC):
**A61K 6/77; A61K 6/836**

(86) International application number:
**PCT/JP2024/007908**

(87) International publication number:
**WO 2024/202945 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.03.2023 JP 2023056115**

(71) Applicants:
• **The University of Osaka**
**Osaka 565-0871 (JP)**
• **GC R&D Corporation**
**Tokyo 174-8585 (JP)**

(72) Inventors:
• **IMAZATO, Satoshi**
**Suita-shi, Osaka 565-0871 (JP)**
• **KITAGAWA, Haruaki**
**Suita-shi, Osaka 565-0871 (JP)**
• **YAMASHITA, Miki**
**Tokyo 174-8585 (JP)**
• **SHOJI, Takumi**
**Tokyo 174-8585 (JP)**
• **AKIYAMA, Ayaka**
**Tokyo 174-8585 (JP)**
• **KONO, Tomoki**
**Tokyo 174-8585 (JP)**

(74) Representative: **Müller-Boré & Partner
Patentanwälte PartG mbB
Friedenheimer Brücke 21
80639 München (DE)**

(54) **DENTAL COMPOSITION AND METHOD FOR PRODUCING DENTAL COMPOSITION**

(57)    A dental composition contains a glass powder complex. The glass powder complex contains a first glass powder, and a second glass powder having dissolvability that is different from dissolvability of the first glass powder in accordance with pH. The first glass powder and the second glass powder both have sustained ion release.

**EP 4 691 449 A1**

**Description**

TECHNICAL FIELD

The present invention relates to a dental composition, and a method for producing a dental composition.

BACKGROUND ART

In the field of dentistry, a technique using sustained ion release glass that sustainably releases functional ions is known. For example, Patent Literature 1 discloses a deodorant composition containing sustained ion release glass that sustainably releases one or more of monovalent to tetravalent ions. Patent Literature 2 discloses a dental hydraulic temporary sealing material composition containing sustained ion release glass that sustainably releases fluoride ions.

CITATION LIST

PATENT LITERATURE

Patent Literature 1: International Publication No. WO2019/189851
Patent Literature 2: Japanese Laid-Open Patent Application Publication No. 2018-95573

SUMMARY OF THE INVENTION

TECHNICAL PROBLEM

Dental compositions containing sustained ion release glass are used in an environment in the oral cavity in which the acidity (pH) is not constant. Thus, when the acidity changes in the oral cavity, the function of sustainably releasing ions cannot be obtained in some cases.

It is an object of the invention of the present application to provide a dental composition that sustainably releases ions in accordance with an environment in the oral cavity.

SOLUTION TO THE PROBLEM

A dental composition according to an aspect of the present invention is a dental composition containing a glass powder complex. The glass powder complex contains a first glass powder, and a second glass powder having dissolvability that is different from dissolvability of the first glass powder in accordance with pH. The first glass powder and the second glass powder both have sustained ion release.

ADVANTAGEOUS EFFECTS OF THE INVENTION

According to an aspect of the present invention, it is possible to provide a dental composition that sustainably releases ions in accordance with an environment in the oral cavity.

DETAILED DESCRIPTION OF THE INVENTION

Hereinafter, embodiments of the present invention will be described in detail. The dental composition according to the present embodiment contains a glass powder complex. In the present specification, the dental composition refers to a composition used in the field of dentistry. The glass powder complex refers to a glass powder containing at least two types of glass.

The glass powder complex contained in the dental composition contains a first glass powder, and a second glass powder having dissolvability that is different from dissolvability of the first glass powder in accordance with pH. The first glass powder and the second glass powder both have sustained ion release.

In the present specification, the term "glass" includes non-crystalline glass, crystalline glass, and glass ceramic containing glass in a part thereof. The glass powder refers to powdered glass. The size of the powder in terms of a median diameter is preferably 0.02 μm or more and 100 μm or less, and more preferably 0.02 μm or more and 30 μm or less.

In the present specification, glass having dissolvability different in accordance with pH indicates that dissolvability of the first glass powder in water and dissolvability of the second glass powder in water are different in accordance with an acidity. Sustained ion release indicates a property in which components contained in glass are dissolved and sustainably released in the state of ions.

No particular limitation is imposed on the components of the first glass powder. A powder of silicate glass is preferably used as the first glass powder. Silicate glass is glass that readily dissolves and readily sustainably releases ions in an acidic range (pH: 6.5 or lower) rather than in a neutral range (pH: higher than 6.5 and lower than 8).

Silicate glass contains silicon (Si), and further contains sodium (Na) and/or potassium (K). Here, silicon plays a role in forming a network in the glass contained in the dental composition.

No particular limitation is imposed on the amount of silicon in the silicate glass, but the amount of silicon in the silicate glass is preferably 15% by mass or more and 70% by mass or less, and more preferably 15% by mass or more and 50% by mass or less, in terms of the amount of silicon oxide ($SiO_2$). When the amount of silicon oxide in the silicate glass is 15% by mass or more, it is possible to readily obtain glass to be contained in a dental composition. When the amount of silicon oxide in the silicate glass is 70% by mass or less, it is possible to readily obtain a dental composition containing glass having a melting temperature that is not excessively high.

No particular limitation is imposed on the amount of sodium and/or potassium in the silicate glass, but the amount of sodium and/or potassium in the silicate glass is preferably 0% by mass or more and 15% by mass or less, and more preferably 1% by mass or more and 10% by mass or less, in terms of the amount of sodium oxide ($Na_2O$) and/or potassium oxide ($K_2O$).

When the silicate glass contains sodium and/or potassium, the melting temperature of the glass contained in the dental composition can be lowered, and the dissolvability of the glass can be further increased. Also, when the amount of sodium oxide and/or potassium oxide in the silicate glass is 15% by mass or less, it is possible to readily obtain glass having a melting temperature that is not excessively high, as glass to be contained in the dental composition.

Specific examples of the silicate glass include soda-lime glass, aluminosilicate glass, borosilicate glass, lead glass, and the like. Of these, soda-lime glass and aluminosilicate glass are preferable because these have high sustained ion release.

The first glass powder, which is a powder of the silicate glass, contains at least one element other than silicon (Si), sodium (Na), and potassium (K). The elements contained in this glass are elements that form ions sustainably released from the glass (hereinafter referred to as sustained release ions).

No particular limitation is imposed on the elements contained in the first glass powder, but examples of the elements contained in the first glass powder include Li, Ca, Sr, Ba, Y, La, Ce, Pr, Nd, Pm, Sm, Eu, Gd, Tb, Dy, Ho, Er, Tm, Yb, Lu, Ti, Zr, Ta, Cu, Ag, Zn, B, Al, Ga, Si, Sn, P, and F. These elements can be contained alone or in combination. Of these, Li, Ca, Sr, Cu, Ag, Zn, B, Ga, and F are preferable.

As the elements contained in the first glass powder, Ca and Sr are preferable in terms of improvement in acid resistance made by the sustained release ions, and Zn is preferable in terms of suppression in acid formation. Also, Ca, Zn, and F are preferable in terms of the tooth demineralization suppressing effect provided by the sustained release ions, and Ca and Sr are preferable in terms of promotion of bone formation. Further, Cu, Ag, Zn, B, Ga, and F are preferable in terms of the antibacterial effect provided by the sustained release ions, and Li is preferable in terms of the anti-inflammatory effect provided by the sustained release ions.

No particular limitation is imposed on the amount of Li contained in the first glass powder, but the amount of Li contained in the first glass powder is preferably 0% by mass or more and 10% by mass or less, and more preferably 1% by mass or more and 8% by mass or less, in terms of the amount of lithium oxide ($LiO_2$). The Li contained in the first glass powder can contribute to impartment of an anti-inflammatory effect. When the amount of Li contained in the first glass powder is 10% by mass or less, it is possible to readily obtain glass having dissolvability in water that is not excessively high, as glass to be contained in the dental composition.

No particular limitation is imposed on the amount of Ca contained in the first glass powder, but the amount of Ca contained in the first glass powder is preferably 0% by mass or more and 40% by mass or less, and more preferably 1% by mass or more and 35% by mass or less, in terms of calcium oxide (CaO). The Ca contained in the first glass powder can contribute to impartment of an acid resistance effect, a tooth demineralization suppressing effect, and a bone formation promoting effect. When the amount of the Ca contained in the first glass powder is 40% by mass or less, it is possible to readily obtain glass to be contained in the dental composition.

No particular limitation is imposed on the amount of Sr contained in the first glass powder, but the amount of Sr contained in the first glass powder is preferably 0% by mass or more and 40% by mass or less, and more preferably 1% by mass or more and 35% by mass or less, in terms of the amount of strontium oxide (SrO). The Sr contained in the first glass powder can contribute to impartment of an acid resistance effect and a bone formation promoting effect. When the amount of the Sr contained in the first glass powder is 40% by mass or less, it is possible to readily obtain a dental composition containing glass having a melting temperature that is not excessively high.

No particular limitation is imposed on the amount of Cu contained in the first glass powder, but the amount of Cu contained in the first glass powder is preferably 0% by mass or more and 10% by mass or less, and more preferably 0.1% by mass or more and 5% by mass or less, in terms of the amount of copper oxide (CuO). The Cu contained in the first glass powder can contribute to impartment of an antibacterial effect. When the amount of Cu contained in the first glass powder is 10% by mass or less, it is possible to readily obtain glass to be contained in the dental composition.

No particular limitation is imposed on the amount of Ag contained in the first glass powder, but the amount of Ag contained in the first glass powder is preferably 0% by mass or more and 5% by mass or less, and more preferably 0.1% by mass or more and 3% by mass or less, in terms of the amount of silver oxide ($Ag_2O$). The Ag contained in the first glass powder can contribute to impartment of an antibacterial effect. When the amount of Ag contained in the first glass powder is 5% by mass or less, it is possible to readily obtain glass to be contained in the dental composition.

No particular limitation is imposed on the amount of Zn contained in the first glass powder, but the amount of Zn contained in the first glass powder is preferably 0% by mass or more and 40% by mass or less, and more preferably 1% by mass or more and 35% by mass or less, in terms of the amount of zinc oxide (ZnO). The Zn contained in the first glass powder can contribute to impartment of an acid formation suppressing effect, a tooth demineralization suppressing effect, and an antibacterial effect. When the amount of Zn contained in the first glass powder is 40% by mass or less, it is possible to readily obtain glass to be contained in the dental composition.

No particular limitation is imposed on the amount of B contained in the first glass powder, but the amount of B contained in the first glass powder is preferably 0% by mass or more and 10% by mass or less, and more preferably 1% by mass or more and 8% by mass or less, in terms of the amount of boron oxide ($B_2O_3$). The B contained in the first glass powder can contribute to impartment of an antibacterial effect. When the amount of B contained in the first glass powder is 10% by mass or less, it is possible to readily obtain a dental composition containing glass in which phase separation is suppressed.

No particular limitation is imposed on the amount of Ga contained in the first glass powder, but the amount of Ga contained in the first glass powder is preferably 0% by mass or more and 40% by mass or less, and more preferably 1% by mass or more and 35% by mass or less, in terms of the amount of gallium oxide ($Ga_2O_3$). The Ga contained in the first glass powder can contribute to impartment of an antibacterial effect. When the amount of Ga contained in the first glass powder is 40% by mass or less, it is possible to readily obtain glass to be contained in the dental composition.

No particular limitation is imposed on the amount of F contained in the first glass powder, but the amount of F contained in the first glass powder is preferably 0% by mass or more and 25% by mass or less, and more preferably 1% by mass or more and 22% by mass or less. The F contained in the first glass powder can contribute to impartment of a tooth demineralization suppressing effect and an antibacterial effect. When the amount of F contained in the first glass powder is 25% by mass or less, it is possible to readily obtain glass to be contained in the dental composition.

No particular limitation is imposed on the amount of the components of the second glass powder. As the second glass powder, a powder of phosphate glass is preferably used. The powder of phosphate glass may be glass that readily dissolves and readily sustainably releases ions in an acidic range (pH: 6.5 or lower) rather than in a neutral range (pH: higher than 6.5 and lower than 8); or may be glass that readily dissolves and readily sustainably releases ions in a neutral range (pH: higher than 6.5 and lower than 8) rather than in an acidic range (pH: 6.5 or lower).

The phosphate glass contains phosphoric acid ($P_2O_5$), and contains sodium (Na) and/or potassium (K). Here, the phosphoric acid plays a role in forming a network in the glass contained in the dental composition.

No particular limitation is imposed on the amount of the phosphoric acid contained in the phosphate glass, but the amount of the phosphoric acid contained in the phosphate glass is preferably 40% by mass or more and 80% by mass or less, and more preferably 40% by mass or more and 70% by mass or less. When the amount of the phosphoric acid contained in the phosphate glass is 40% by mass or more, it is possible to readily obtain glass to be contained in the dental composition. When the amount of the phosphoric acid contained in the phosphate glass is 80% by mass or less, it is possible to readily obtain a dental composition containing glass having dissolvability in water that is not excessively high.

No particular limitation is imposed on the amount of sodium and/or potassium contained in the phosphate glass, but the amount of sodium and/or potassium contained in the phosphate glass is preferably 5% by mass or more and 30% by mass or less, and more preferably 5% by mass or more and 20% by mass or less, in terms of the amounts of sodium oxide ($Na_2O$) and potassium oxide ($K_2O$).

When the amount of sodium and/or potassium contained in the phosphate glass is 5% by mass or more, it is possible to lower the melting temperature of the glass contained in the dental composition, and further increase the dissolvability of the glass. Also, when the amount of sodium and/or potassium contained in the phosphate glass is 30% by mass or less, it is possible to readily obtain glass having dissolvability in water that is not excessively high, as glass to be contained in the dental composition.

The second glass powder, which is a powder of the phosphate glass, contains at least one element other than phosphorus (P), sodium (Na), and potassium (K). The elements contained in this glass are elements that form the sustained release ions.

No particular limitation is imposed on the elements contained in the second glass powder, but examples of the elements contained in the second glass powder include Li, Ca, Sr, Ba, Y, La, Ce, Pr, Nd, Pm, Sm, Eu, Gd, Tb, Dy, Ho, Er, Tm, Yb, Lu, Ti, Zr, Ta, Cu, Ag, Zn, B, Al, Ga, Si, Sn, P, and F. These elements can be contained alone or in combination. Of these, Li, Ca, Sr, Cu, Ag, Zn, B, Ga, and F are preferable.

As the elements contained in the second glass powder, Ca and Sr are preferable in terms of improvement in acid resistance made by the sustained release ions, and Zn is preferable in terms of suppression in acid formation. Ca, Zn, and F are preferable in terms of the tooth demineralization suppressing effect provided by the sustained release ions, and Ca and Sr are preferable in terms of promotion of bone formation. Further, Cu, Ag, Zn, B, Ga, and F are preferable in terms of the antibacterial effect provided by the sustained release ions, and Li is preferable in terms of the anti-inflammatory effect provided by the sustained release ions.

No particular limitation is imposed on the amount of Li contained in the second glass powder, but the amount of Li contained in the second glass powder is preferably 0% by mass or more and 20% by mass or less, and more preferably 1% by mass or more and 15% by mass or less, in terms of the amount of lithium oxide ($LiO_2$). The Li contained in the second glass powder can contribute to impartment of an anti-inflammatory effect. When the amount of Li contained in the second glass powder is 20% by mass or less, it is possible to readily obtain glass having dissolvability in water that is not excessively high, as glass to be contained in the dental composition.

No particular limitation is imposed on the amount of Ca contained in the second glass powder, but the amount of Ca contained in the second glass powder is preferably 0% by mass or more and 40% by mass or less, and more preferably 1% by mass or more and 35% by mass or less, in terms of the amount of calcium oxide (CaO). The Ca contained in the second glass powder can contribute to impartment of an acid resistance effect, a tooth demineralization suppressing effect, and a bone formation promoting effect. When the amount of Ca contained in the second glass powder is 40% by mass or less, it is possible to readily obtain glass to be contained in the dental composition.

No particular limitation is imposed on the amount of Sr contained in the second glass powder, but the amount of Sr contained in the second glass powder is preferably 0% by mass or more and 40% by mass or less, and more preferably 1% by mass or more and 35% by mass or less, in terms of the amount of strontium oxide (SrO). The Sr contained in the second glass powder can contribute to impartment of an acid resistance effect and an osteogenesis promoting effect. When the amount of Sr contained in the second glass powder is 40% by mass or less, it is possible to readily obtain a dental composition containing glass having a melting temperature that is not excessively high.

No particular limitation is imposed on the amount of Cu contained in the second glass powder, but the amount of Cu contained in the second glass powder is preferably 0% by mass or more and 10% by mass or less, and more preferably 0.1% by mass or more and 5% by mass or less, in terms of the amount of copper oxide (CuO). The Cu contained in the second glass powder can contribute to impartment of an antibacterial effect. When the amount of Cu contained in the second glass powder is 10% by mass or less, it is possible to readily obtain glass to be contained in the dental composition.

No particular limitation is imposed on the amount of Ag contained in the second glass powder, but the amount of Ag contained in the second glass powder is preferably 0% by mass or more and 5% by mass or less, and more preferably 0.1% by mass or more and 3% by mass or less, in terms of the amount of silver oxide ($Ag_2O$). The Ag contained in the second glass powder can contribute to impartment of an antibacterial effect. When the amount of Ag contained in the second glass powder is 5% by mass or less, it is possible to readily obtain glass to be contained in the dental composition.

No particular limitation is imposed on the amount of Zn contained in the second glass powder, but the amount of Zn contained in the second glass powder is preferably 0% by mass or more and 20% by mass or less, and more preferably 1% by mass or more and 15% by mass or less, in terms of the amount of zinc oxide (ZnO). The Zn contained in the second glass powder can contribute to impartment of an acid formation suppressing effect, a tooth demineralization suppressing effect, and an antibacterial effect. When the amount of Zn contained in the second glass powder is 20% by mass or less, it is possible to readily obtain glass to be contained in the dental composition.

No particular limitation is imposed on the amount of B contained in the second glass powder, but the amount of B contained in the second glass powder is preferably 0% by mass or more and 10% by mass or less, and more preferably 1% by mass or more and 8% by mass or less, in terms of the amount of boron oxide ($B_2O_3$). The B contained in the second glass powder can contribute to impartment of an antibacterial effect. When the amount of B contained in the second glass powder is 10% by mass or less, it is possible to readily obtain a dental composition containing glass in which phase separation is suppressed.

No particular limitation is imposed on the amount of Ga contained in the second glass powder, but the amount of Ga contained in the second glass powder is preferably 0% by mass or more and 25% by mass or less, and more preferably 1% by mass or more and 20% by mass or less, in terms of the amount of gallium oxide ($Ga_2O_3$). The Ga contained in the second glass powder can contribute to impartment of an antibacterial effect. When the amount of Ga contained in the second glass is 25% by mass or less, it is possible to readily obtain glass to be contained in the dental composition.

No particular limitation is imposed on the amount of F contained in the second glass powder, but the amount of F contained in the second glass powder is preferably 0% by mass or more and 20% by mass or less, and more preferably 1% by mass or more and 15% by mass or less. The F contained in the second glass powder can contribute to impartment of a tooth demineralization suppressing effect and an antibacterial effect. When the amount of F contained in the second glass powder is 20% by mass or less, it is possible to readily obtain glass to be contained in the dental composition.

No particular limitation is imposed on a ratio by mass of the first glass powder versus the second glass powder contained in the glass powder complex. For example, the ratio by mass of the first glass powder versus the second glass powder is 10:1 to 1:10, preferably 7:1 to 1:7, and more preferably 5:1 to 1:5. When the ratio by mass of the first glass powder versus the second glass powder contained in the glass powder complex is 10:1 to 1:10, a change in the sustained release amount of the sustained release ions can be controlled in accordance with a change in acidity.

The dental composition of the present embodiment may optionally contain other components unless such optional components do not impair the object of the present invention. Examples of the other components that can be contained include: polymers, such as polyacrylic acid and the like; curing accelerators, such as hydrochlorides, sulfates, and the like; oily components, such as hydrocarbons, higher fatty acids, esters, and the like; various inorganic or organic colorants; antibacterial agents; perfumes; and the like.

In the dental composition of the present embodiment, at least one element contained in the first glass powder may be the same as at least one element contained in the second glass powder. For example, when zinc (Zn) is contained in the first glass powder, zinc (Zn) is also contained in the second glass powder.

In the dental composition of the present embodiment, at least one element contained in the first glass powder may be different from the element contained in the second glass powder, and at least one element contained in the second glass powder may be different from the element contained in the first glass powder. For example, when zinc (Zn) is contained in the first glass powder but strontium (Sr) is not contained in the first glass powder, zinc (Zn) is not contained in the second glass powder but strontium (Sr) is contained in the second glass powder.

In the dental composition of the present embodiment, a dissolution rate of the second glass powder in the neutral range is preferably higher than a dissolution rate of the second glass powder in the acidic range.

The dissolution rate is calculated according to the following formula after dipping glass in an acetic acid-sodium acetate buffer solution.

$$\text{Dissolution rate (\%)} = [(\text{Weight before dipping}) - (\text{Weight after dipping})/\text{Weight before dipping}] \times 100$$

In the present embodiment, as described above, the glass powder complex contained in the dental composition contains the first glass powder and the second glass powder having dissolvability different in accordance with pH, and the first glass powder and the second glass powder both have sustained ion release. Thus, the sustained release amount of the sustained release ions can be changed in accordance with the change in acidity in the oral cavity.

For example, the dental composition contains, as the glass powder complex, a glass powder complex in which the first glass powder is a powder of silicate glass that sustainably releases zinc ions ($Zn^{2+}$) and the second glass powder is a powder of phosphate glass that sustainably releases zinc ions ($Zn^{2+}$). In this case, even if the acidity in the oral cavity changes, the amount of the sustained release ions released from the dental composition can be maintained or controlled in accordance with that change.

Specifically, according to this dental composition, zinc ions ($Zn^{2+}$) are readily sustainably released from the first glass powder contained in the glass powder complex when the acidity in the oral cavity is acidic (e.g., pH 4.5), while the zinc ions ($Zn^{2+}$) are not readily sustainably released when the acidity in the oral cavity is neutral (e.g., pH 7.5). On the other hand, zinc ions ($Zn^{2+}$) are readily sustainably released from the second glass powder contained in the glass powder complex both when the acidity in the oral cavity is acidic (e.g., pH 4.5) and when the acidity in the oral cavity is neutral (e.g., pH 7.5).

As a result, both when the acidity in the oral cavity is acidic and when the acidity in the oral cavity is neutral, zinc ions ($Zn^{2+}$) are released from the dental composition as the sustained release ions, thereby maintaining the sustained release amount of the sustained release ions in the oral cavity. Therefore, even if the acidity in the oral cavity changes, the antibacterial effect, the acid formation suppressing effect, and the tooth demineralization suppressing effect, which are provided by zinc ions ($Zn^{2+}$), can be maintained in the oral cavity.

In the present embodiment, by using, in the glass powder complex contained in the dental composition, a silicate glass powder as the first glass powder and a phosphate glass powder as the second glass powder, a remarkable effect of changing the sustained release amount of the sustained release ions in accordance with the change in acidity can be obtained

In the present embodiment, as described above, the first glass powder and the second glass powder both contain at least one element selected from Li, Ca, Sr, Ga, Cu, Zn, B, and F. Thus, in the present embodiment, in the case of Li, the sustained release ions can have an anti-inflammatory effect. In the case of Ca, the tooth demineralization suppressing effect can be obtained, the acid resistance can be improved, and the bone formation can be promoted. In the case of Sr, the acid resistance can be improved, and the bone formation can be promoted.

In the present embodiment, in the case of Cu and Ag, the antibacterial effect can be imparted to the dental composition. In the case of Zn, the antibacterial effect, the acid formation suppressing effect, and the tooth demineralization suppressing effect can be imparted to the dental composition. In the case of B and Ga, the antibacterial effect can be obtained. In the case of F, the antibacterial effect and the tooth demineralization suppressing effect are obtained.

In the dental composition of the present embodiment, as described above, at least one element contained in the first glass powder is the same as at least one element contained in the second glass powder, and thus even if the acidity in the oral cavity changes, the same sustained release ions can be sustainably released in the oral cavity, thereby enabling maintenance of the sustained release amount.

For example, when zinc (Zn) is contained in both the first glass powder and the second glass powder, the sustained release amount of zinc ions ($Zn^{2+}$) can be maintained or increased/decreased even if the environment in which zinc ions ($Zn^{2+}$) are sustainably released changes from the acidic range to the neutral range.

In the present embodiment, as described above, at least one element contained in the first glass powder is different from the element contained in the second glass powder and at least one element contained in the second glass powder is different from the element contained in the first glass powder, and thus different sustained release ions can be sustainably released in the oral cavity in accordance with the change of acidity.

In the dental composition of the present embodiment, as described above, the dissolution rate of the second glass powder in the neutral range is higher than the dissolution rate of the second glass powder in the acidic range. Thus, in the present embodiment, even if the dissolution rate of the first glass powder in the neutral range is lower than the dissolution rate of the first glass powder in the acidic range, the sustained release ions are sustainably released from the second glass powder in the neutral range, and thus even if the acidity changes from acidic to neutral, the sustained release amount of the sustained release ions can be maintained.

The dental composition of the present embodiment can be used for various dental materials by virtue of the above-described effects. No particular limitation is imposed on applications of the dental composition of the present embodiment, but examples of the applications include dental cements, dental adhesives (dental bonding materials), dental temporary sealing materials, dental temporary attachment materials, dental primers, dental coating agents, root covering materials, dental composite resins, dental hard resins, dental cutting resin materials, dental temporary restoration materials, dental fillers, toothpastes, and the like.

The dental composition of the present embodiment may contain other components in accordance with applications. Examples of the other components include polymers, polymerization initiators, polymerization inhibitors, and the like. For example, an ionomer cement can be formed by mixing a polymer, such as polyacrylic acid or the like, with the glass powder complex.

The polymer usable may be formed of a polymerizable monomer. Examples of the polymerizable monomer include, for example, homopolymers or copolymers of a (meth)acrylate compound. In the present specification, the (meth) acrylate refers to at least one selected from an acrylate and a methacrylate.

In the present specification, the homopolymer refers to a polymer mainly formed of a constituent unit of a certain polymerization component. The copolymer refers to a polymer in which a constituent unit of a certain polymerization component is copolymerized with a constituent unit of another polymerization component. The homopolymer and the copolymer may contain other polymerization components that are unavoidably included.

Examples of the (meth)acrylate compound include glycerol dimethacrylate (GDMA), bisphenol A diglycidyl methacrylate (Bis-GMA), urethane dimethacrylate (UDMA, di-2-methacryloyloxyethyl-2,2,4-trimethylhexamethylene dicarbamate), 2,2-bis(4-methacryloyloxyethoxyphenyl)propane (Bis-MEPP), tricyclodecanedimethanol dimethacrylate (DCP), triethylene glycol dimethacrylate (TEGDMA), neopentyl glycol dimethacrylate (NPG), and the like. These (meth) acrylate compounds may be used alone or in combination.

The amount of the homopolymer or copolymer of the (meth)acrylate compound contained in the dental composition of the present embodiment is preferably, for example, 3% by mass or more and 45% by mass or less, more preferably 4% by mass or more and 40% by mass or less, and further preferably 5% by mass or more and 30% by mass or less.

No particular limitation is imposed on the polymerization initiator, but examples of the polymerization initiator include camphorquinone (CQ), ethyl p-dimethylaminobenzoate (EPA), (2,4,6-trimethylbenzoyl)diphenylphosphine oxide (TPO), 2-(2-hydroxy-5-methylphenyl)benzotriazole, and the like.

No particular limitation is imposed on the amount of the polymerization initiator contained in the dental composition, but the amount of the polymerization initiator contained in the dental composition is, for example, 0.01% by mass or more and 5% by mass or less, preferably 0.03% by mass or more and 2% by mass or less, and more preferably 0.05% by mass or more and 2% by mass or less.

No particular limitation is imposed on the polymerization inhibitor, but examples of the polymerization inhibitor include 6-tert-butyl-2,4-xylenol, 2,6-di-tert-butyl-p-cresol, and the like.

No particular limitation is imposed on the amount of the polymerization inhibitor contained in the dental composition, but the amount of the polymerization inhibitor contained in the dental composition is, for example, 0.005% by mass or more and 5% by mass or less, preferably 0.01% by mass or more and 3% by mass or less, and more preferably 0.03% by mass or more and 1% by mass or less.

A method for producing a dental composition according to the present embodiment is substantially a method for producing the dental composition containing the above-described glass powder complex. Specifically, the method includes a step of mixing the first glass powder and the second glass powder having dissolvability different in accordance with pH, the first glass powder and the second glass powder both having sustained ion release.

When a polymer is contained in the dental composition, a monomer liquid is prepared by mixing a polymerizable monomer constituting the polymer, along with a polymerization initiator, a polymerization inhibitor, and the like.

In the step of mixing the first glass powder and the second glass powder in the method for producing the dental composition according to the present embodiment, the first glass powder and the second glass powder contained in the above-described glass powder complex are used. Therefore, the glass powder complex obtained by the method for producing the dental composition according to the present embodiment has the effects of the above-described glass powder complex.

That is, the obtained dental composition is a dental composition in which the glass powder complex contained in the dental composition contains the first glass powder and the second glass powder having dissolvability different in accordance with pH, and the first glass powder and the second glass powder both have sustained ion release. Therefore, according to the method for producing the dental composition according to the present embodiment, it is possible to obtain a dental composition in which the sustained release amount of the sustained release ions can change in accordance with the change in acidity in the oral cavity.

EXAMPLES

Hereinafter, the present invention will be described in more detail by way of Examples. Various tests and evaluations are performed according to the following methods.

<Silicate Glass>

Raw materials of silicate glass were weighed, and mixed in a mortar for 10 minutes. The resulting mixture was charged into a platinum crucible, and melted at 1,350°C for 1 hour. The resulting melt was cooled in water to undergo vitrification. The obtained glass was recovered, dried at 110°C for 5 hours, and milled in a planetary mill (15-mm alumina balls, 150 rpm) for 30 minutes to 1 hour, thereby obtaining glass components S1 to S5. Also, a commercially available quartz glass filler (hereinafter referred to as QG) was used.

<Phosphate Glass>

Raw materials of phosphate glass were weighed, and mixed in a mortar for 10 minutes. The resulting mixture was charged into a platinum crucible, and melted at 1,100°C for 1 hour. The resulting melt was cooled in an iron press. This was milled in a ball mill for 30 minutes (ethanol wet milling, 40-mm alumina balls, 100 rpm) and further milled in a ball mill for 30 minutes (ethanol wet milling, 5-mm alumina balls, 100 rpm). Subsequently, the glass powder was recovered through centrifugation, and dried under reduced pressure (-0.1 MPa, 40°C) to evaporate the remaining ethanol, thereby obtaining glass components PN1, PN2, PA1, and PA2.

<Glass Composition>

The glass powder (molded with a polyvinyl chloride ring) was analyzed using an X-ray fluorescence analyzer (ZSX Primus IV, obtained from Rigaku Corporation), thereby determining the compositions of the glass powder. Tables 1 and 2 show the evaluation results of the compositions (unit: % by mass) of the glass powder.

<Particle Size Distribution>

Using a laser diffraction/scattering particle size distribution analyzer (Partica LA-960V2, obtained from Horiba Ltd.), the silicate glass was dispersed in distilled water for measurement, and the phosphate glass was dispersed in ethanol for measurement. The particle sizes of the glass shown in Tables 1 to 4 were confirmed to be $10\pm2~\mu$m in terms of D50 (median diameter).

<Dissolution Amount (Glass Alone)>

0.1 g of the glass powder was charged into 10 ml of an acetic acid-sodium acetate buffer solution of pH 4.5 or 10 ml of a hydroxyethylpiperazineethanesulfonic acid (HEPES) buffer solution of pH 7.5, followed by storing at 37°C for one day while being stirred at 10 rpm. Subsequently, filtration through a glass filter sheet was performed, and the dissolution rate was calculated from the weight of the residue according to the following formula.

Dissolution rate (%) = [(Weight before dipping) - (Weight after dipping)/Weight before dipping] $\times$ 100

Table 1 shows the glass compositions and the dissolution rates of the silicate glass (glass components S1 to S5 and QG), and Table 2 shows the glass compositions and the dissolution rates of the phosphate glass (glass components PN1, PN2, PA1, and PA2).

[Table 1]

| Glass composition/% by mass | | | S1 | S2 | S3 | S4 | S5 | QG |
|---|---|---|---|---|---|---|---|---|
| $P_2O_5$ | | | - | - | - | 6.2 | 8.2 | - |
| $Na_2O$ | | | 6.7 | 6.6 | 6.5 | 5.6 | 9.4 | - |
| CaO | | | 9.7 | 3.5 | - | 9.8 | 9.0 | - |
| $SiO_2$ | | | 38.7 | 24.5 | 24.3 | 24.7 | 24.9 | 100 |
| SrO | | | - | - | - | - | - | - |
| $Al_2O_3$ | | | 7.2 | - | - | 31.8 | 27.8 | - |
| ZnO | | | 34.4 | 30.3 | 35.0 | - | - | - |
| $Ga_2O_3$ | | | - | - | - | - | - | - |
| $La_2O_3$ | | | - | 31.8 | 31.2 | - | - | - |
| F | | | 3.3 | 3.3 | 3.0 | 21.9 | 20.7 | - |
| pH 4.5 | Dissolution rate (%) | | 11 | 21 | 24 | 12.4 | 4.9 | 0 |
| | Ion elution amount (ppm) | $Zn^{2+}$ | 533 | 564 | 567 | - | - | - |
| | | $Ca^{2+}$ | - | - | - | 113 | 57 | - |
| | | $Sr^{2+}$ | - | - | - | - | - | - |
| | | $Ga^{2+}$ | - | - | - | - | - | - |
| | | $F^-$ | 66 | 2 | 2 | 111 | 111 | - |
| pH 7.5 | Dissolution rate (%) | | 5 | 6 | 4 | 4.6 | 4.2 | 0 |
| | Ion elution amount (ppm) | $Zn^{2+}$ | 8 | 10 | 10 | - | - | - |
| | | $Ca^{2+}$ | - | - | - | 10 | 11 | - |
| | | $Sr^{2+}$ | - | - | - | - | - | - |
| | | $Ga^{3+}$ | - | - | - | - | - | - |
| | | $F^-$ | 7 | 3 | 2 | 12 | 20 | - |

[Table 2]

| Glass composition/% by mass | PN1 | PN2 | PA1 | PA2 |
|---|---|---|---|---|
| $P_2O_5$ | 65.1 | 55.7 | 54.8 | 54.6 |
| $Na_2O$ | 14.8 | 9.5 | 7.8 | 6.6 |
| CaO | - | - | - | - |
| $SiO_2$ | - | - | - | - |
| SrO | - | 34.8 | 33.9 | 33.3 |
| $Al_2O_3$ | 3.9 | - | 3.5 | 5.5 |
| ZnO | - | - | - | - |
| $Ga_2O_3$ | 16.2 | - | - | - |
| $La_2O_3$ | - | - | - | - |
| F | - | - | - | - |

(continued)

| Glass composition/% by mass | | | PN1 | PN2 | PA1 | PA2 |
|---|---|---|---|---|---|---|
| pH 4.5 | Dissolution rate (%) | | 29 | 45 | 15 | 17 |
| | Ion elution amount (ppm) | $Zn^{2+}$ | - | - | - | - |
| | | $Ca^{2+}$ | - | - | - | - |
| | | $Sr^{2+}$ | - | 958 | 464 | 373 |
| | | $Ga^{2+}$ | 185 | - | - | - |
| | | $F^-$ | - | - | - | - |
| pH 7.5 | Dissolution rate (%) | | 40 | 61 | 5 | 6 |
| | Ion elution amount (ppm) | $Zn^{2+}$ | - | - | - | - |
| | | $Ca^{2+}$ | - | - | - | - |
| | | $Sr^{2+}$ | - | 1684 | 66 | 53 |
| | | $Ga^{3+}$ | 427 | - | - | - |
| | | $F^-$ | - | - | - | - |
| pH range in which dissolution rate is higher | | | Acidic< Neutral | Acidic< Neutral | Acidic> Neutral | Acidic> Neutral |

<Ion Elution Amount of Glass Alone>

0.1 g of the glass powder was charged into 10 ml of an acetic acid-sodium acetate buffer solution of pH 4.5 or 10 ml of a HEPES buffer solution of pH 7.5, followed by storing at 37°C for one day while being stirred at 10 rpm. Subsequently, centrifugation was performed twice at 2,000 rpm for 10 minutes, followed by filtration through a 0.2-$\mu$m membrane filter. The resulting filtrate was measured for the concentrations of $Zn^{2+}$, $Sr^{2+}$, $Ca^{2+}$, and $Ga^{3+}$ using an inductively coupled plasma optical emission spectrometer (ICP-OES) (iCAP 7200 Duo, obtained from Thermo Fisher Scientific, Inc.), and the concentration of $F^-$ was measured using a fluorine electrode. Table 1 shows the ion elution amounts of the silicate glass, and Table 2 shows the ion elution amounts of the phosphate glass.

<Ion Elution Amount of Glass Powder Complex>

Two types of glass powder (at optional amounts) were each charged into 10 ml of an acetic acid-sodium acetate buffer solution of pH 4.5 or 10 ml of a HEPES buffer solution of pH 7.5, followed by storing at 37°C for one day while being stirred at 10 rpm. Subsequently, centrifugation was performed twice at 2,000 rpm for 10 minutes, followed by filtration through a 0.2-$\mu$m membrane filter. The resulting filtrate was measured for the concentrations of $Zn^{2+}$, $Sr^{2+}$, $Ca^{2+}$, and $Ga^{3+}$ using the ICP-OES, and the concentration of $F^-$ was measured using a fluorine electrode.
Table 3 shows the compositions of the glass powder complexes and the ion elution amounts of Examples 1 to 5, and Table 4 shows the compositions of the glass powder complexes and the ion elution amounts of Comparative Examples 1 to 5.

[Table 3]

|  | | | Ex. 1 | Ex. 2 | Ex. 3 | Ex. 4 | Ex. 5 |
|---|---|---|---|---|---|---|---|
| Composition (g) | Silicate-based glass | S1 | - | 0.1 | - | - | - |
|  |  | S2 | - | - | 0.1 | - | - |
|  |  | S3 | 0.1 | - | - | - | - |
|  |  | S4 | - | - | - | 0.1 | - |
|  |  | S5 | - | - | - | - | 0.1 |
|  | Non-dissolvable glass | QG | - | - | - | - | - |
|  | Phosphate-based glass | PN1 | 0.5 | 0.1 | - | 0.1 | - |
|  |  | PN2 | - | - | 0.1 | - | 0.1 |
|  |  | PA1 | - | - | - | - | - |
|  |  | PA2 | - | - | - | - | - |
| Ion elution amount (ppm) | pH 4.5 | $Zn^{2+}$ | 625 | 431 | 497 | - | - |
|  |  | $Ca^{2+}$ | - | 95 | 56 | 124 | 33 |
|  |  | $Sr^{2+}$ | - | - | 40 | - | 81 |
|  |  | $Ga^{3+}$ | 0 | 1 | - | 0 | - |
|  |  | $F^-$ | 8 | 48 | 2 | 146 | 61 |
|  | pH 7.5 | $Zn^{2+}$ | 281 | 66 | 110 | - | - |
|  |  | $Ca^{2+}$ | - | 13 | 14 | 12 | 9 |
|  |  | $Sr^{2+}$ | - | - | 525 | - | 1228 |
|  |  | $Ga^{3+}$ | 166 | 43 | - | 367 | - |
|  |  | $F^-$ | 17 | 14 | 18 | 12 | 14 |

[Table 4]

|  | | | Comp. Ex. 1 | Comp. Ex. 2 | Comp. Ex. 3 | Comp. Ex. 4 | Comp. Ex. 5 |
|---|---|---|---|---|---|---|---|
| Composition (g) | Silicate-based glass | S1 | - | - | - | - | - |
|  |  | S2 | - | - | - | - | - |
|  |  | S3 | - | 0.1 | - | - | - |
|  |  | S4 | - | - | - | - | - |
|  |  | S5 | - | - | 0.1 | - | - |
|  | Non-dissolvable glass | QG | - | - | - | - | 0.1 |
|  | Phosphate-based glass | PN1 | - | - | - | - | - |
|  |  | PN2 | 0.1 | - | - | 0.1 | 0.1 |
|  |  | PA1 | 0.1 | - | 0.1 | - | - |
|  |  | PA2 | - | 0.9 | - | 0.1 | - |

(continued)

| | | | Comp. Ex. 1 | Comp. Ex. 2 | Comp. Ex. 3 | Comp. Ex. 4 | Comp. Ex. 5 |
|---|---|---|---|---|---|---|---|
| Ion elution amount (ppm) | pH 4.5 | $Zn^{2+}$ | - | 445 | - | - | - |
| | | $Ca^{2+}$ | - | 225 | 134 | - | - |
| | | $Sr^{2+}$ | 305 | - | 53 | 832 | 950 |
| | | $Ga^{3+}$ | 72 | - | - | - | - |
| | | $F^-$ | - | - | 132 | - | - |
| | pH 7.5 | $Zn^{2+}$ | - | 14 | - | - | - |
| | | $Ca^{2+}$ | - | 33 | 66 | - | - |
| | | $Sr^{2+}$ | 540 | - | 15 | 1759 | 1681 |
| | | $Ga^{3+}$ | 214 | - | - | - | - |
| | | $F^-$ | - | - | 7 | - | - |

<Bonding Material and Composite Resin>

Glass alone and a glass powder complex were prepared by the above-described method as an example of the dental composition according to the present invention, assuming a bonding material or a composite resin. Table 5 shows the glass compositions and the dissolution rates of silicate glass (a glass component S6 and a commercially available particulate silica, RX50), and Table 6 shows the glass compositions and the dissolution rates of phosphate glass (glass components PA3 and PA4).

[Table 5]

| Glass composition/% by mass | | | S6 | RX50 |
|---|---|---|---|---|
| $P_2O_5$ | | | - | - |
| $Na_2O$ | | | 6.2 | - |
| $SiO_2$ | | | 21.1 | 100 |
| $SrO$ | | | - | - |
| $Al_2O_3$ | | | - | - |
| $ZnO$ | | | 39.4 | - |
| $Ga_2O_3$ | | | - | - |
| $La_2O_3$ | | | 29.6 | - |
| $F$ | | | 3.7 | - |
| Median diameter ($\mu$m) | | | 0.3 | - |
| pH 4.5 | Dissolution rate (%) | | 18 | 0 |
| | Ion elution amount (ppm) | $Zn^{2+}$ | 677 | - |
| | | $Sr^{2+}$ | - | - |
| | | $Ga^{2+}$ | - | - |
| | | $F^-$ | 2 | - |
| pH 7.5 | Dissolution rate (%) | | 5 | 0 |
| | Ion elution amount (ppm) | $Zn^{2+}$ | 12 | - |
| | | $Sr^{2+}$ | - | - |
| | | $Ga^{2+}$ | - | - |
| | | $F^-$ | 7 | - |

[Table 6]

| Glass composition/% by mass | | | PN3 | PN4 |
|---|---|---|---|---|
| $P_2O_5$ | | | 55.7 | 65.1 |
| $Na_2O$ | | | 9.5 | 14.8 |
| $SiO_2$ | | | - | - |
| SrO | | | 34.8 | - |
| $Al_2O_3$ | | | - | 3.9 |
| ZnO | | | - | - |
| $Ga_2O_3$ | | | - | 16.2 |
| $La_2O_3$ | | | - | - |
| F | | | - | - |
| Median diameter ($\mu$m) | | | 8.8 | 3.4 |
| pH 4.5 | Dissolution rate (%) | | 45 | 58 |
| | Ion elution amount (ppm) | $Zn^{2+}$ | - | - |
| | | $Sr^{2+}$ | 958 | - |
| | | $Ga^{2+}$ | - | 471 |
| | | $F^-$ | - | - |
| pH 7.5 | Dissolution rate (%) | | 61 | 84 |
| | Ion elution amount (ppm) | $Zn^{2+}$ | - | - |
| | | $Sr^{2+}$ | 1684 | - |
| | | $Ga^{2+}$ | - | 753 |
| | | $F^-$ | - | - |
| pH range in which dissolution rate is higher | | | Acidic< Neutral | Acidic< Neutral |

<Ion Elution Amounts of Bonding Material and Composite Resin>

The glass powder complex prepared assuming the bonding material and the composite resin was charged into a ring having a diameter of 10 mm and a thickness of 2 mm. Using a light irradiator (G-Light Prima-II Plus, obtained from GC CORPORATION), light irradiation was performed on the front and rear surfaces at 9 points for 10 seconds each, thereby curing the composition. A surface, i.e., a test surface, of the obtained cured body was polished using a #1200-SiC waterproof polishing sheet. The cured body was dipped in 3 mL of an acetic acid-sodium acetate buffer solution of pH 4.5 or 3 mL of a HEPES buffer solution of pH 7.5, followed by storing at 37°C for one day while being stirred at 10 rpm. The filtrate obtained through a 0.2-$\mu$m membrane filter was measured for the concentrations of $Zn^{2+}$, $Sr^{2+}$, $Ca^{2+}$, and $Ga^{3+}$ using the ICP-OES, and the concentration of $F^-$ was measured using a fluorine electrode.

Table 7 shows the compositions of the bonding materials (Examples 1-1 and 1-2 and Comparative Examples 1-1 and 1-2) and the ion elution amounts, and Table 8 shows the compositions of the composite resins (Examples 2-1 to 2-4 and Comparative Example 2-1) and the ion elution amounts.

[Table 7]

| Form | | | Ex. 1-1 | Ex. 1-2 | Comp. Ex. 1-1 | Comp. Ex. 1-2 |
|---|---|---|---|---|---|---|
| | | | Bonding material | | | |
| Monomer liquid | Polymerizable monomer | Bis-GMA | 11.3 | 11.3 | 14.3 | 11.3 |
| | | UDMA | 23.7 | 23.7 | 29.6 | 23.7 |
| | | GDMA | 35.1 | 35.1 | 43.8 | 35.1 |
| | | Bis-MEPP | - | - | - | - |
| | | DCP | - | - | - | - |
| | | TEGDMA | - | - | - | - |
| | | NPG | - | - | - | - |
| | Polymerization initiator | CQ | 0.9 | 0.9 | 1.1 | 0.9 |
| | | EPA | 1.7 | 1.7 | 2.2 | 1.7 |
| | | TPO | 2.7 | 2.7 | 3.3 | 2.7 |
| | | TINUVIN P | - | - | - | - |
| | Polymerization inhibitor | IA | - | - | - | - |
| | | BHT | 0.5 | 0.5 | 0.6 | 0.5 |
| Other fillers | Silica | RX50 | 4.1 | 4.1 | 5.1 | 4.1 |
| | | SO-C2 | - | - | - | - |
| Sustained ion release glass | Silicate-based glass | S6 | 10 | 10 | - | - |
| | Phosphate-based glass | PN3 | 10 | - | - | 20 |
| | | PN4 | - | 10 | - | - |
| Ion release amount (ppm) | pH 4.5 | $Zn^{2+}$ | 2.9 | 5.0 | - | - |
| | | $Ga^{3+}$ | - | 0.0 | - | - |
| | | $Sr^{2+}$ | 3.0 | - | - | 5.6 |
| | | $F^-$ | 0.4 | 0.5 | - | - |
| | pH 7.5 | $Zn^{2+}$ | 1.6 | 1.1 | - | - |
| | | $Ga^{3+}$ | - | 1.6 | - | - |
| | | $Sr^{2+}$ | 4.3 | - | - | 8.5 |
| | | $F^-$ | 0.5 | 0.5 | - | - |

[Table 8]

| Form | | | Ex. 2-1 | Ex. 2-2 | Ex. 2-3 | Ex. 2-4 | Comp. Ex. 2-1 |
|---|---|---|---|---|---|---|---|
| Form | | | Composite resin | | | | |
| Monomer liquid | Polymerizable monomer | Bis-GMA | - | - | - | - | - |
| | | UDMA | - | - | 7.8 | 6.8 | - |
| | | GDMA | - | - | - | - | - |
| | | Bis-MEPP | 27.0 | 27.0 | 27.4 | 24 | 27.0 |
| | | DCP | 6.6 | 6.6 | - | - | 6.6 |
| | | TEGDMA | - | - | 3.9 | 3.4 | - |
| | | NPG | 5.0 | 5.0 | - | - | 5.0 |
| | Polymerization initiator | CQ | 0.15 | 0.15 | 0.2 | 0.17 | 0.15 |
| | | EPA | 0.28 | 0.28 | 0.4 | 0.34 | 0.28 |
| | | TPO | 0.46 | 0.46 | 0.04 | 0.04 | 0.46 |
| | | TINUVIN P | 0.2 | 0.2 | 0.13 | 0.12 | 0.2 |
| | Polymerization inhibitor | IA | 0.04 | 0.04 | 0.13 | 0.13 | 0.04 |
| | | BHT | 0.27 | 0.27 | - | - | 0.27 |
| Other fillers | Silica | RX50 | - | - | - | - | - |
| | | SO-C2 | - | - | - | - | 60 |
| Sustained ion release glass | Silicate-based glass | S6 | 30 | 30 | 30 | 32.5 | - |
| | Phosphate-based glass | PN3 | 30 | - | - | - | - |
| | | PN4 | - | 30 | 30 | 32.5 | - |
| Ion release amount (ppm) | pH 4.5 | $Zn^{2+}$ | 47.2 | 43.5 | 45.8 | 62.5 | - |
| | | $Ga^{3+}$ | - | 0.7 | 0.0 | 0.0 | - |
| | | $Sr^{2+}$ | 48.1 | - | - | - | - |
| | | F - | 1.2 | 0.9 | 1.0 | 1.1 | - |
| | pH 7.5 | $Zn^{2+}$ | 11.5 | 9.0 | 9.9 | 10.6 | - |
| | | $Ga^{3+}$ | - | 9.6 | 8.5 | 10.3 | - |
| | | $Sr^{2+}$ | 32.0 | - | - | - | - |
| | | F - | 1.5 | 1.3 | 1.7 | 2.2 | - |

Abbreviations in Tables 7 and 8 are as follows.

Bis-GMA: Bisphenol A diglycidyl methacrylate
UDMA: Urethane dimethacrylate [di-2-methacryloyloxyethyl-2,2,4-trimethylhexamethylene dicarbamate]
GDMA: Glycerol dimethacrylate
Bis-MEPP: 2,2-bis(4-methacryloyloxyethoxyphenyl)propane
DCP: Tricyclodecanedimethanol dimethacrylate
TEGDMA: Triethylene glycol dimethacrylate
NPG: Neopentyl glycol dimethacrylate
CQ: Camphorquinone
EPA: Ethyl p-dimethylaminobenzoate
TPO: (2,4,6-Trimethylbenzoyl)diphenylphosphine oxide
TINUBIN P: 2-(2-Hydroxy-5-methylphenyl)benzotriazole (TINUVIN (registered trademark) P, obtained from BASF)
IA: 6-tert-Butyl-2,4-xylenol
BHT: 2,6-di-tert-Butyl-p-cresol
RX50: Particulate silica surface-treated with hexamethyldisilazane (AEROSIL (registered trademark) RX50, obtained from NIPPON AEROSIL CO., LTD.)
SO-C2: Silica (ADMAFINE (registered trademark) SO-C2, obtained from Admatechs)

From Tables 1, 2, and 3, in each of Examples 1 to 5, the ion elution amount of at least one type of ions in the acidic range is different, i.e., greater or smaller, than the ion elution amount of the at least one type of ions in the neutral range, indicating acidity (pH) responsiveness. Conversely, from Tables 1, 2, and 4, in each of Comparative Examples 1 to 5, the elution amounts of the ions in the acidic range are all greater or smaller than the elution amounts of these ions in the neutral range, indicating no acidity (pH) responsiveness.

From Tables 5 to 8, in each of the Examples of the bonding materials (Examples 1-1 and 1-2) and the Examples of the composite resins (Examples 2-1 to 2-4), the ion elution amount of at least one type of ions in the acidic range is different, i.e., greater or smaller, than the ion elution amount of the at least one type of ion in the neutral range, indicating acidity (pH) responsiveness. Conversely, in each of the Comparative Examples of the bonding materials (Comparative Examples 1-1 to 1-3) and the Comparative Example of the composite resin (Comparative Example 2-1), the elution amounts of the ions in the acidic range are all greater or smaller than the elution amounts of the ions in the neutral range, indicating no acidity (pH) responsiveness.

It has been found based on these results that the glass powder complex contained in the dental composition contains the first glass powder and the second glass powder having dissolvability different in accordance with pH, and the first glass powder and the second glass powder both have sustained ion release, and thus the sustained release amounts of the sustained release ions can be changed in accordance with the changes in acidity in the oral cavity.

The above-disclosed embodiments include, for example, the following aspects.

(Clause 1) A dental composition, including:

a glass powder complex, wherein
the glass powder complex contains

a first glass powder, and
a second glass powder having dissolvability that is different from dissolvability of the first glass powder in accordance with pH, and

the first glass powder and the second glass powder both have sustained ion release.

(Clause 2) The dental composition according to clause 1, wherein

the first glass powder is a powder of silicate glass, and
the second glass powder is phosphate glass.

(Clause 3) The dental composition according to clause 1 or 2, wherein
the first glass powder and the second glass powder both contain at least one element selected from Li, Ca, Sr, Cu, Ag, Zn, B, Ga, and F.

(Clause 4) The dental composition according to any one of clauses 1 to 3, wherein
at least one element contained in the first glass powder is same as at least one element contained in the second glass powder.

(Clause 5) The dental composition according to any one of clauses 1 to 4, wherein

at least one element contained in the first glass powder is different from an element contained in the second glass powder, and
at least one element contained in the second glass powder is different from an element contained in the first glass powder.

(Clause 6) The dental composition according to clause 5, wherein
a dissolution rate of the second glass powder in a neutral range is higher than a dissolution rate of the second glass powder in an acidic range.

(Clause 7) A method for producing a dental composition containing a glass powder complex, the method including:

a step of mixing a first glass powder, and a second glass powder having dissolvability that is different from dissolvability of the first glass powder in accordance with pH, wherein
the first glass powder and the second glass powder both have sustained ion release.

Although the embodiments of the present invention have been described above, the present invention is not limited to the specific embodiments, and various alterations and modifications are possible within the scope of the invention recited in claims.

The present application claims priority to Japanese Patent Application No. 2023-056115, filed on March 30, 2023, the entire contents of which are incorporated herein by reference.

**Claims**

1.  A dental composition, comprising:

    a glass powder complex, wherein
    the glass powder complex contains

       a first glass powder, and
       a second glass powder having dissolvability that is different from dissolvability of the first glass powder in accordance with pH, and

    the first glass powder and the second glass powder both have sustained ion release.

2.  The dental composition according to claim 1, wherein

    the first glass powder is a powder of silicate glass, and
    the second glass powder is phosphate glass.

3.  The dental composition according to claim 1, wherein
    the first glass powder and the second glass powder both contain at least one element selected from Li, Ca, Sr, Cu, Ag, Zn, B, Ga, and F.

4.  The dental composition according to claim 1, wherein
    at least one element contained in the first glass powder is same as at least one element contained in the second glass powder.

5.  The dental composition according to any one of claims 1 to 4, wherein

    at least one element contained in the first glass powder is different from an element contained in the second glass powder, and
    at least one element contained in the second glass powder is different from an element contained in the first glass powder.

6.  The dental composition according to claim 5, wherein
    a dissolution rate of the second glass powder in a neutral range is higher than a dissolution rate of the second glass powder in an acidic range.

7.  A method for producing a dental composition containing a glass powder complex, the method comprising:

    a step of mixing a first glass powder, and a second glass powder having dissolvability that is different from dissolvability of the first glass powder in accordance with pH, wherein
    the first glass powder and the second glass powder both have sustained ion release.

# INTERNATIONAL SEARCH REPORT

| International application No. |
|---|
| **PCT/JP2024/007908** |

## A.    CLASSIFICATION OF SUBJECT MATTER

*A61K 6/77*(2020.01)i; *A61K 6/836*(2020.01)i
FI:    A61K6/77; A61K6/836

According to International Patent Classification (IPC) or to both national classification and IPC

## B.    FIELDS SEARCHED

Minimum documentation searched (classification system followed by classification symbols)

A61K6/77; A61K6/836

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

## C.    DOCUMENTS CONSIDERED TO BE RELEVANT

| Category* | Citation of document, with indication, where appropriate, of the relevant passages | Relevant to claim No. |
|---|---|---|
| Y | WO 2019/069564 A1 (GC CORP.) 11 April 2019 (2019-04-11) claims 1, 2, paragraphs [0007]-[0012], [0020], [0027]-[0028], [0042], [0055], [0075], table 1, examples 1-4 | 1-7 |
| Y | WO 2018/012352 A1 (GC CORP.) 18 January 2018 (2018-01-18) claims 1, 2, paragraphs [0006]-[0016], [0035]-[0036], [0042]-[0043], [0059]-[0064], [0067], table 1, examples 1-8 | 1-7 |
| P, X | JP 2023-97144 A (OSAKA UNIVERSITY) 07 July 2023 (2023-07-07) claims 1-7, paragraphs [0058]-[0065] | 1-7 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 May 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/007908**

| Patent document<br>cited in search report | | | Publication date<br>(day/month/year) | Patent family member(s) | Publication date<br>(day/month/year) |
|---|---|---|---|---|---|
| WO | 2019/069564 | A1 | 11 April 2019 | US 2020/0276089 A1<br>claims 1, 2, paragraphs [0008]-[0014], [0022], [0029]-[0031], [0045]-[0046], [0059], [0089]-[0090], table 1, examples 1-4 | |
| WO | 2018/012352 | A1 | 18 January 2018 | US 2019/0151204 A1<br>claims 1, 2, paragraphs [0008]-[0020], [0038]-[0039], [0045]-[0047], [0063]-[0073], [0076], table 1, examples 1-8<br>US 2021/0070651 A1<br>EP 3485868 A1<br>JP 2020-152730 A | |
| JP | 2023-97144 | A | 07 July 2023 | US 2023/0202909 A1<br>claims 1-7, paragraphs [0063]-[0070]<br>EP 4201899 A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019189851 A
- JP 2018095573 A

- JP 2023056115 A